# EUROPÄISCHE PATENTSCHRIFT

(11) **EP 0 495 228 B1**
(45) Veröffentlichungstag und Bekanntmachung des Hinweises auf die Patenterteilung: **22.03.1995**
(21) Anmeldenummer: 91121908.7
(22) Anmeldetag: 20.12.1991
(51) Int. Cl.: C08G 73/14, C07D 209/48

(54) **N-Substituierte Polyamidimide**
Substituted polyamide-imides
Polyamide-imides substitués

(30) Priorität: 18.01.1991 DE 4101379
(43) Veröffentlichungstag der Anmeldung: 22.07.1992
(73) Patentinhaber: BASF Aktiengesellschaft, 67063 Ludwigshafen (DE)
(72) Erfinder: Wolf, Peter, Dr., W-6710 Frankenthal (DE); Koch, Juergen, Dr., W-6708 Neuhofen (DE)

(56) Entgegenhaltungen:
- US-A- 3 904 582

## Beschreibung

Die Erfindung betrifft in organischen Lösungsmitteln lösliche sowie schmelzbare Polyamidimide mit sehr guter Hitzebeständigkeit und hoher Glasübergangstemperatur sowie ein Verfahren zu ihrer Herstellung.

Aromatische Polyamidimide besitzen im allgemeinen eine sehr gute thermische und chemische Beständigkeit. Sie finden deshalb Anwendung in Technologien, die hohe Temperaturstabilität erfordern, wie etwa in der Elektronik oder der Luftfahrt. Polyamidimide werden dabei als Klebstoffe, Formkörper, Fasern, Filme, Verbundwerkstoffe usw. eingesetzt.

Der Nachteil der bekannten Polyamidimide ist die im Verlauf ihrer Synthese und Verarbeitung auftretende Wasserentwicklung, die auf Imidisierungs- und Kettenverlängerungsreaktionen sowie eine hohe Feuchtigkeitsaufnahmefähigkeit zurückzuführen ist. Weiterhin ist die Löslichkeit der Polyamidimide in vielen organischen Lösungsmitteln sehr gering, so daß eine Verarbeitung aus Lösungen solcher Polyamidimide vielfach nicht möglich ist.

Eine Aufgabe ist daher die Schaffung neuer hochtemperaturbeständiger Kunststoffe, die die oben angegebenen Vorteile besitzen, dabei jedoch eine möglichst geringe Wassermenge unter den Synthese- und Verarbeitungsbedingungen freisetzen und im Vergleich zu konventionellen Polyamidimiden gesteigerte Löslichkeit in organischen Solvenzien besitzen.

Die Erfindung löst die Aufgabe durch neue polymere Verbindungen, die als Kettenbausteine sowohl solche vom Imidtyp als auch solche von N-substituierten Amiden besitzen und insbesondere dadurch, daß die verwendeten Monomerbausteine in bereits vollständig imidisierter Form eingesetzt werden. Dadurch entfällt bei der Herstellung der erfindungsgemäßen Polyamidimide die bisher beobachtete Wasserentwicklung durch Imidisierungsreaktionen.

Überraschenderweise ist die Löslichkeit der erfindungsgemäßen Polyamidimide vielfach wesentlich besser als bisher bekannter Polyamidimide.

Unmittelbarer Erfindungsgegenstand sind lösliche und/oder schmelzbare, am Amidstickstoff substituierte Polyamidimide mit einer Glasübergangstemperatur oberhalb von 150°C und gegebenenfalls einer Schmelztemperatur von bis zu 450°C, die im wesentlichen aus wiederkehrenden Einheiten der allgemeinen Formel I aufgebaut sind: 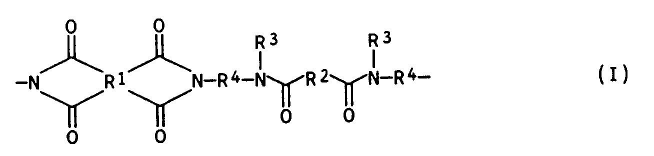
wobei R¹ ein tetravalentes aromatisches Radikal, ausgewählt aus einer der nachstehenden Strukturen sowie deren Substitutionsprodukten 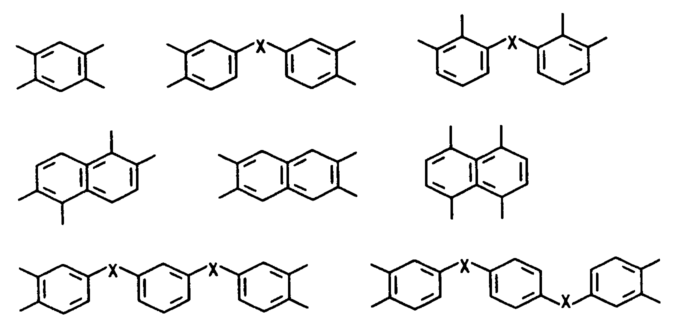
und X eine direkte Bindung bedeutet oder ein Bindeglied, ausgewählt aus 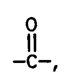
-O-, -S-, -SO₂-, -CH₂-, =C(CH₃)₂, =C(CF₃)₂, =Si(CH₃)₂
In der vorstehenden Formel (I) stehen die Reste R² und R⁴ unabhängig voneinander für ein divalentes aromatisches Radikal, ausgewählt aus einer der Strukturen 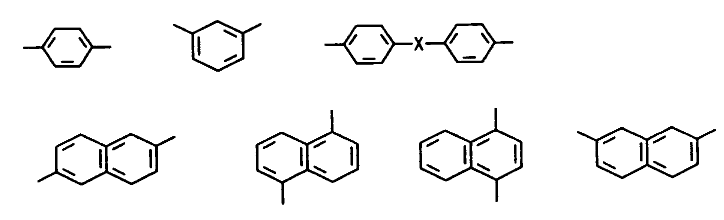
wobei X die vorstehend angegebene Bedeutung besitzt.

Schließlich steht in vorstehender Formel (I) der Rest R³ für ein monovalentes Radikal wie C₁-C₆-Alkyl und Phenyl sowie deren C₁-C₆-Alkyl-, C₁-C₆-Alkoxy-, Aryl- oder halogensubstituierte Derivate. Ein gewisser Anteil aller Reste R³ - z.B. bis zu 50 % - kann Wasserstoff sein.

Die neuen Polymeren erhält man vorteilhaft durch die Polykondensation eines entsprechenden Bis-phthalimids der Struktur (II) mit einem entsprechenden Dicarbonsäurehalogenid 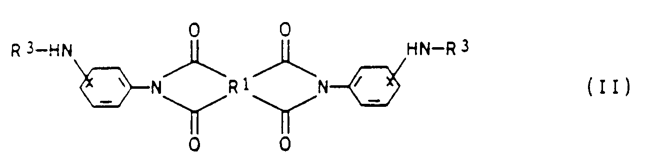
Die Bausteine (Monomeren) der Struktur (II), die an sich nicht bekannt sind, können z.B. durch die Umsetzung von N-monosubstituierten Phenylendiaminen der Struktur 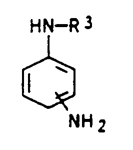
mit Tetracarbonsäuredianhydriden in prinzipiell bekannter Weise erhalten werden.

### Beispiele für solche Monomere (II) sind etwa

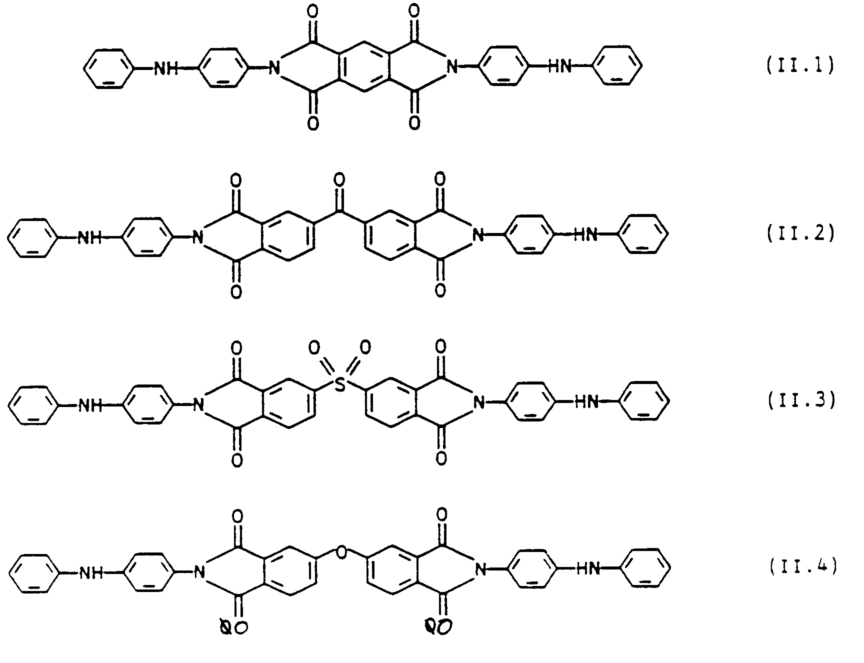

Ersetzt man in diesem Verfahren die Monomeren (II) teilweise durch primäre Diamine der allgemeinen Struktur H₂N-R-NH₂, wobei R für ein beliebiges divalentes organisches Radikal steht, so können auch Co-Polyamidimide hergestellt werden, die lediglich eine partielle Alkylierung bzw. Phenylierung am Amidstickstoff aufweisen (d.h. bei denen ein Teil aller Reste R³ Wasserstoff ist).

Ein alternatives Verfahren zur Synthese der Polyamidimide (I) bildet die Umsetzung von N-monosubstituierten Phenylendiaminen mit einem Tetracarbonsäuredianhydrid und anschließende Umsetzung des erhaltenen Zwischenproduktes mit einem Dicarbonsäuredichlorid. Die Synthese kann auf diese Weise als Eintopf-Verfahren durchgeführt werden.

Die Polykondensation wird in polaren, aprotischen Lösungsmitteln durchgeführt, die einen Siedepunkt von mehr als 100°C, vorzugsweise mehr als 150°C besitzen. Beispiele für verwendbare Lösungsmittel sind N,N-Dimethylacetamid (DMA), N-Methylpyrrolidon (NMP), N-Methylcaprolactam, Dimethylsulfoxid (DMSO), Sulfolan, Diphenylsulfon, Nitrobenzol, Anisol oder Diphenylether. Die Umsetzung wird bei einer Temperatur zwischen etwa 120°C und 300°C durchgeführt, die von den Reaktionsteilnehmern und dem gewählten Lösungsmittel abhängt.

Der Feststoffgehalt der Lösungen beträgt dabei i.a. 10 bis 50 %. Der Reaktionsdauer richtet sich nach gewünschtem Kondensationsgrad sowie nach der Reaktivität der eingesetzten Monomeren, liegt im allgemeinen zwischen 2 und 12 Stunden. Im Anschluß an die Polykondensation können zur Stabilisierung etwa vorhandene freie Aminoendgruppen mit einem monofunktionellen Acylierungsmittel, wie z.B. Benzoylchlorid, umgesetzt werden.

Die Umsetzung wird im allgemeinen unter einem Inertgas, wie Stickstoff oder Argon durchgeführt.

Die Umsetzung kann in Gegenwart einer Base aus der Gruppe der tertiären Amine, wie z.B. Pyridin, Chinolin, Isochinolin oder Tri-tert.Butylamin durchgeführt werden.

### Herstellung der monomeren Bis(phthalimide) (II.1) bis (II.4)

Die Monomeren werden auf im Prinzip bekannte Weise, z.B. durch Umsetzung eines Mol-Äquivalents des betreffenden Tetracarbonsäuredianhydrides mit zwei Mol.-Äquivalenten 4-Aminodiphenylamin erhalten.

### Herstellung der N-substituierten Polyamidimide (I)

### Beispiel 1

### Polykondensation des Bis(phthalimids) II.2 mit Isophthaloylchlorid

32,73 g (50 mmol) des Bis(phthalimids) II.2 werden mit 130 g Diphenylsulfon und 10,15 g (50 mmol) Isophthaloylchlorid vermischt. Die feste Mischung wird unter einer Stickstoffatmosphäre auf 140°C erhitzt, wobei das Diphenylsulfon aufschmilzt. Man rührt eine Stunde bei dieser Temperatur, erhöht die Temperatur auf 170°C und läßt eine weitere Stunde rühren. Schließlich wird die Temperatur auf 200°C gesteigert und man rührt noch vier Stunden. Man gießt die viskose Mischung auf eine Metallplatte, zerkleinert den gebildeten Feststoff und entfernt das Diphenylsulfon durch Extraktion mit heißem Methanol. Man erhält 38,6 g (98,3 %) Polymer der Struktur I.1 als gelblichen Feststoff und mit einer reduzierten Viskosität von 53 ml/g (gemessen in NMP). Das Polymer besitzt eine Glasübergangstemperatur von 243°C, es ist löslich in Methylenchlorid, Chloroform, DMA und NMP und unlöslich in Aceton.

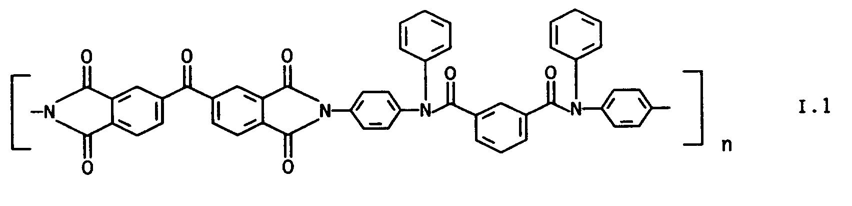

### Beispiel 2

### Polykondensation des Bis(phthalimids) II.2 mit Terephthaloylchlorid

32,73 g (50 mmol) des Bis(phthalimids) II.2 werden mit 130 g Diphenylsulfon und 10,15 g (50 mmol) Terephthaloylchlorid vermischt. Die feste Mischung wird unter einer Stickstoffatmosphäre auf 140°C erhitzt, wobei das Diphenylsulfon aufschmilzt. Man rührt eine Stunde bei dieser Temperatur, erhöht die Temperatur für eine Stunde auf 170°C und schließlich für weitere vier Stunden auf 200°C. Nach dem Abkühlen zerkleinert man den gebildeten Feststoff und entfernt das Diphenylsulfon durch Extraktion mit heißem Methanol. Man erhält 38,2 g (97,3 %) Polymer der Struktur I.2 als gelblichen Feststoff und mit einer reduzierten Viskosität von 42 ml/g (gemessen in m-Kresol). Das Polymer besitzt eine Glasübergangstemperatur von 246°C und eine Schmelztemperatur von 388°C.

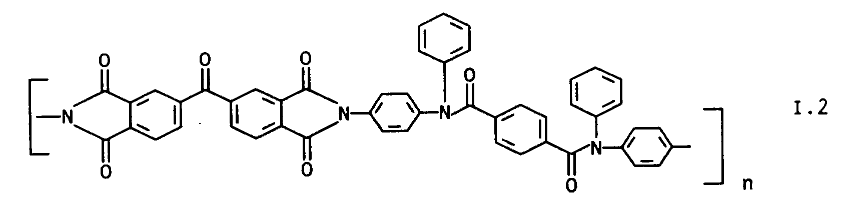

### Beispiel 3

### Polykondensation des Bis(phthalimids) II.3 mit Isophthaloylchlorid

34,54 g (50 mmol) des Bis(phthalimids) II.3 werden mit 150 ml NMP suspendiert. Anschließend werden 10,15 g (50 mmol) Isophthaloylchlorid zugesetzt. Die Mischung wird unter einer Stickstoffatmosphäre auf 140°C erhitzt, wobei langsam eine klare Lösung entsteht. Man rührt eine Stunde bei dieser Temperatur, erhöht die Temperatur dann auf 170°C und läßt eine weitere Stunde rühren. Schließlich wird die Temperatur auf 200°C gesteigert und man rührt noch vier Stunden. Man fällt das erhaltene Produkt in 600 ml Methanol, saugt den gebildeten gelblichen Feststoff ab, wäscht gründlich mit Methanol und trocknet im Vakuum bei 150°C. Man erhält 40,6 g (98,9 %) Polymer der Struktur I.3 als gelblichen Feststoff und mit einer reduzierten Viskosität von 51 ml/g. Das Polymer besitzt eine Glasübergangstemperatur von 247°C.

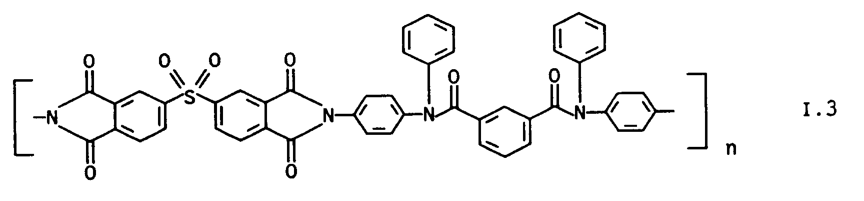

### Beispiel 4

### Polykondensation des Bis(phthalimids) II.3 mit Terephthaloylchlorid

34,54 g (50 mmol) des Bis(phthalimids) II.3 werden mit 150 g NMP suspendiert. Anschließend werden 10,15 g (50 mmol) Terephthaloylchlorid zugesetzt. Die Mischung wird unter einer Stickstoffatmosphäre auf 140°C erhitzt, wobei langsam eine klare Lösung entsteht. Man rührt eine Stunde bei dieser Temperatur, erhöht die Temperatur auf 170°C und läßt eine weitere Stunde rühren. Schließlich wird die Temperatur auf 200°C gesteigert und die Mischung für weitere vier Stunden bei dieser Temperatur gerührt. Man fällt das erhaltene Produkt in 600 ml Methanol, saugt den gebildeten gelblichen Feststoff ab, wäscht gründlich mit Methanol und trocknet im Vakuum bei 150°C. Man erhält 40,6 g (98,9 %) Polymer der Struktur I.4 als gelblichen Feststoff und mit einer reduzierten Viskosität von 62 ml/g. Das Polymer besitzt eine Glasübergangstemperatur von 230°C.

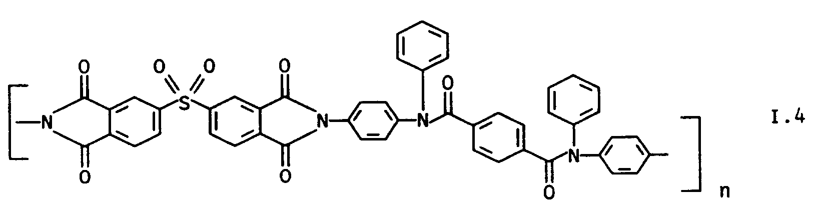

### Beispiel 5

### Polykondensation von 4-Aminodiphenylamin mit 5,5'-Sulfonyl-diphthalsäuredianhydrid und Isophthaloylchlorid (Eintopf-Verfahren)

22,1 g (120 mmol) 4-Aminodiphenylamin werden in 200 ml NMP unter Stickstoff gelöst. Man kühlt die Mischung mit einem Eisbad auf 0 bis 5°C und setzt dann 21,49 g (60 mmol) 5,5'-Sulfonyl-diphthalsäuredianhydrid zu. Die Lösung wird 20 Minuten unter Kühlung gerührt; dann wird das Eisbad entfernt und man läßt weitere 40 Minuten bei Raumtemperatur rühren. Anschließend gibt man 40 ml Toluol und 10 ml Isochinolin zu und erhitzt am Wasserabscheider auf 140 bis 145°C Innentemperatur. Man rührt bei dieser Temperatur, bis sich kein Wasser mehr abscheidet. Dann gibt man 12,18 g (60 mmol) Isophthaloylchlorid zu und läßt eine weitere Stunde rühren. Das Toluol wird dann aus der Mischung abdestilliert, bis die Temperatur im Kolben über 170°C zu steigen beginnt. Man rührt noch eine Stunde bei dieser Temperatur und schließlich weitere vier Stunden bei 200°C. Anschließend läßt man abkühlen und fällt das Reaktionsprodukt Mischung in 600 ml Methanol. Der erhaltene Feststoff wird mit Methanol gewaschen. Man trocknet das Produkt bei 150°C im Vakuum. Man erhält 40,3 g (98,2 %) Polymer der Struktur I.3 als gelblichen Feststoff und mit einer reduzierten Viskosität von 49 ml/g.

### Beispiel 6

### Polykondensation des Bis(phthalimids) II.1 mit Isophthaloylchlorid

Die Umsetzung wird durchgeführt wie in Beispiel 3 beschrieben. Das resultierende Polyamidimid besitzt eine reduzierte Viskosität von 43 ml/g und eine Glasübergangstemperatur von 251°C.

### Beispiel 7

### Polykondensation des Bis(phthalimids) II.1 mit Terephthaloylchlorid

Die Umsetzung wird durchgeführt wie in Beispiel 3 beschrieben. Das resultierende Polyamidimid besitzt eine reduzierte Viskosität von 46 ml/g, eine Glasübergangstemperatur von 262°C und eine Schmelztemperatur von 489°C.

### Beispiel 8

### Polykondensation des Bis(phthalimids) II.4 mit Isophthaloylchlorid

Die Umsetzung wird durchgeführt wie in Beispiel 3 beschrieben. Das resultierende Polyamidimid besitzt eine reduzierende Viskosität von 59 ml/g und eine Glasübergangstemperatur von 237°C.

## Patentansprüche

1. N-Substituiertes Polyamidimid, aufgebaut im wesentlichen aus identischen oder verschiedenen wiederkehrenden Einheiten der allgemeinen Formel I 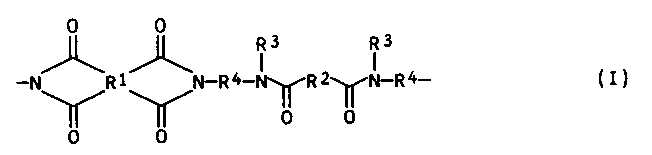 wobei R¹ ein tetravalentes aromatisches Radikal, ausgewählt aus einer der Strukturen 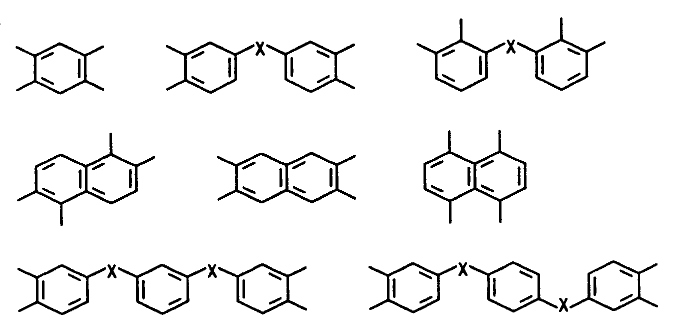 und X eine direkte Bindung bedeutet oder ein Bindeglied, ausgewählt aus 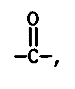 -O-, -S-, -SO₂-, -CH₂-, =C(CH₃)₂, =C(CF₃)₂, =Si(CH₃)₂;
wobei R² und R⁴ unabhängig voneinander für ein divalentes aromatisches Radikal stehen, ausgewählt aus einer der Strukturen 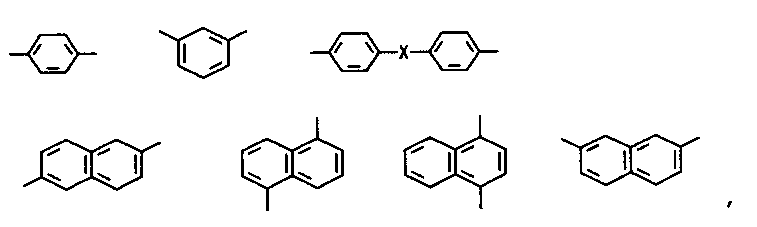 wobei X die vorstehend angegebene Bedeutung besitzt und wobei R³ für Wasserstoff oder ein monovalentes Radikal wie C₁-C₆-Alkyl und Phenyl sowie deren C₁-C₆-Alkyl-, C₁-C₆-Alkoxy-, Aryl- oder halogensubstituierte Derivate steht, mit der Maßgabe, daß nicht mehr als 50 % aller Reste R³ gleichzeitig Wasserstoff sind.

2. Polyamidimid nach Anspruch 1, wobei R², R³ und R⁴ für Phenylen, Phenyl bzw. p-Phenylen stehen.

3. Partiell N-substituiertes Polyamidimid aus 10 bis 100 mol-% Einheiten der Struktur (I) nach Anspruch 1 und bis zu 90 mol-% wiederkehrenden Einheiten der allgemeinen Formel 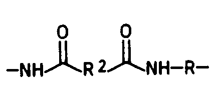 wobei R für ein beliebiges divalentes organisches Radikal steht.

4. Bis(phthalimid) der Struktur 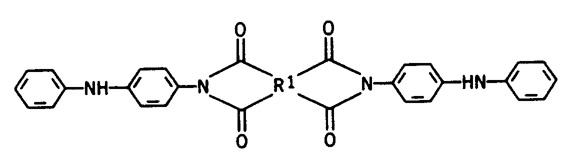 wobei R¹ die in Anspruch 1 angegebene Bedeutung hat und wobei für R¹ die Struktur 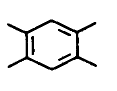 ausgenommen ist.

5. Verfahren zur Herstellung von Polyamidimiden nach Anspruch 1, dadurch gekennzeichnet, daß ein entsprechendes Bis(phthalimid) mit einem entsprechenden Dicarbonsäuredichlorid in einem polaren, aprotischen Lösungsmittel bei einer Temperatur zwischen 120 und 300°C polykondensiert wird.

6. Verfahren zur Herstellung von Polyamidimiden nach Anspruch 1, dadurch gekennzeichnet, daß in einem Eintopfprozess ein 4-Aminophenylalkylamin oder ein 4-Aminophenylarylamin mit einem entsprechenden Tetracarbonsäuredianhydrid in einem polaren, aprotischen Lösungsmittel umgesetzt und das erhaltene Zwischenprodukt mit einem entsprechenden Dicarbonsäuredichlorid polykondensiert wird, wobei die Reaktionstemperatur zwischen 120 und 300°C liegt.

7. Verfahren nach Anspruch 5, dadurch gekennzeichnet, daß die Polykondensation in Gegenwart einer Base aus der Gruppe der tertiären Amine durchgeführt wird.

## Claims

1. An N-substituted polyamide-imide essentially composed of identical or different repeating units of the formula I 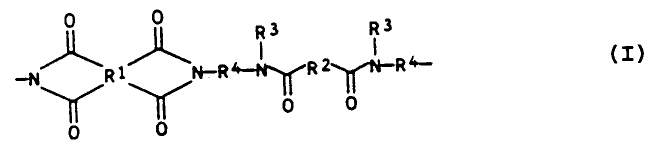 where R¹ is one of the following tetravalent aromatic radicals 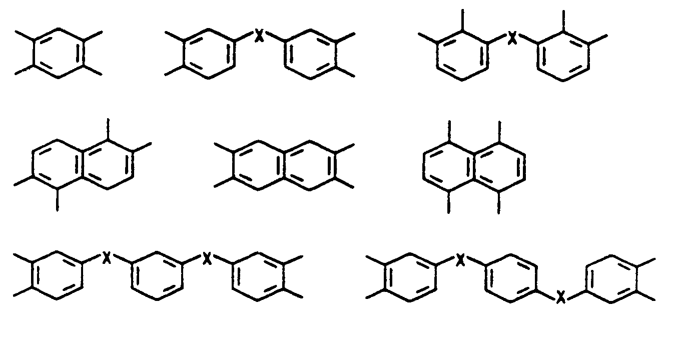 and X is a bond or one of the following linkers 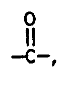 -O-, -S-, -SO₂-, -CH₂-, =C(CH₃)₂, =C(CF₃)₂, =Si(CH₃)₂;
where R² and R⁴ are each, independently of one another, one of the following divalent aromatic radicals 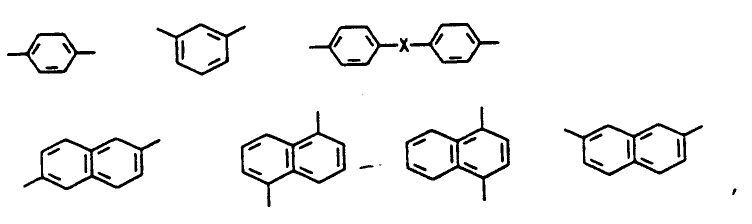 and where X has the abovementioned meanings and where R³ is hydrogen or a monovalent radical such as C₁-C₆-alkyl or phenyl, and the C₁-C₆-alkyl-, C₁-C₆-alkoxy-, aryl- or halogen-substituted derivatives thereof, with the proviso that not more than 50% of all R³ radicals are hydrogen at any one time.

2. A polyamide-imide as claimed in claim 1, where R², R³ and R⁴ are phenylene, phenyl and p-phenylene respectively.

3. A partially N-substituted polyamide-imide composed of from 10 to 100 mol% of units of the structure (I) as claimed in claim 1 and of up to 90 mol% of repeating units of the formula 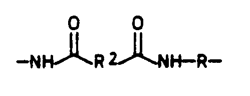 where R is any divalent organic radical.

4. A bis(phthalimide) of the structure 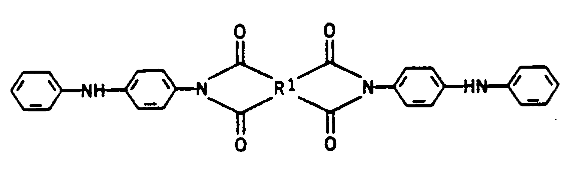 where R¹ has the meanings indicated in claim 1 except

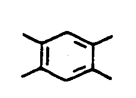

5. A process for preparing a polyamide-imide as claimed in claim 1, which comprises polycondensation of a corresponding bis(phthalimide) with a corresponding dicarbonyl dichloride in a polar aprotic solvent at from 120 to 300°C.

6. A process for preparing a polyamide-imide as claimed in claim 1, which comprises a one-pot process in which a 4-aminophenylalkylamine or a 4-aminophenylarylamine is reacted with an appropriate tetracarboxylic dianhydride in a polar aprotic solvent, and the resulting intermediate undergoes polycondensation with an appropriate dicarbonyl dichloride at from 120 to 300°C.

7. A process as claimed in claim 5, wherein the polycondensation is carried out in the presence of a tertiary amine.

## Revendications

1. Polyamide-imide N-substitué, constitué essentiellement par des motifs répétitifs identiques ou différents de formule générale I 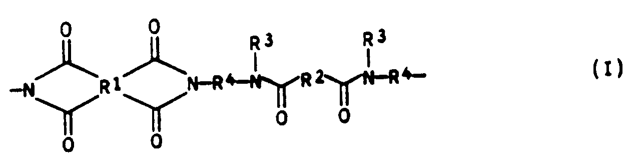 dans laquelle R¹ représente un radical aromatique tétravalent, choisi parmi l'une des structures 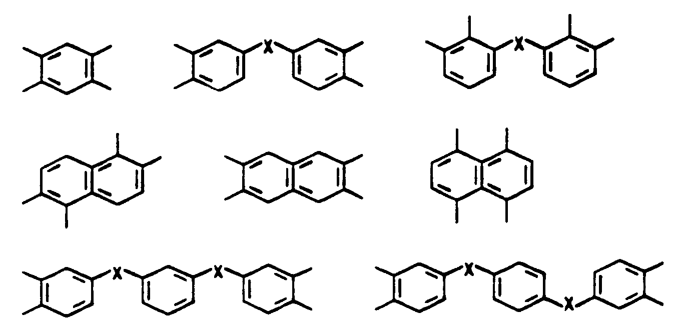 X désignant une liaison directe, ou un maillon de liaison choisi parmi 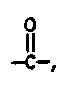 -O-, -S-, -SO₂-, -CH₂-, =C(CH₃)₂, =C(CF₃)₂, =Si(CH₃)₂;
R² et R⁴ sont mis chacun, indépendamment l'un de l'autre, pour un radical aromatique divalent, choisi parmi l'une des structures 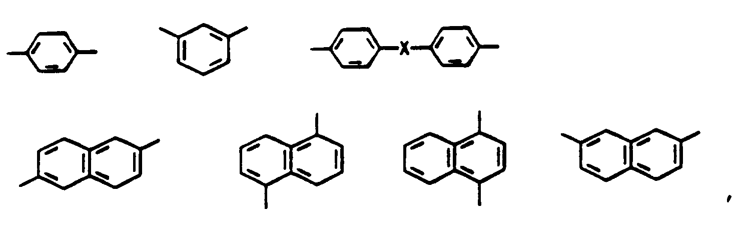 X ayant la signification susmentionnée, et R³ est mis pour un atome d'hydrogène ou pour un radical monovalent tel qu'un radical alkyle en C₁-C₆ ou phényle, ainsi que leurs dérivés substitués par des restes alkyle en C₁-C₆, alcoxy en C₁-C₆, aryle ou par des atomes d'halogène, étant spécifié que pas plus de 50% de tous les restes R³ ne sont en même temps des atomes d'hydrogène.

2. Polyamide-imide selon la revendication 1, dans lequel R², R³ et R⁴ sont mis chacun pour un radical phénylène, phényle ou p-phénylène.

3. Polyamide-imide partiellement N-substitué, constitué par 10 à 100% en moles de motifs de structure (I) selon la revendication 1 et par 90% en moles au maximum de motifs répétitifs de formule générale 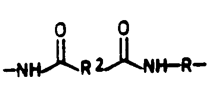 dans laquelle R est mis pour un radical organique divalent quelconque.

4. Bis(phtalimide) de structure 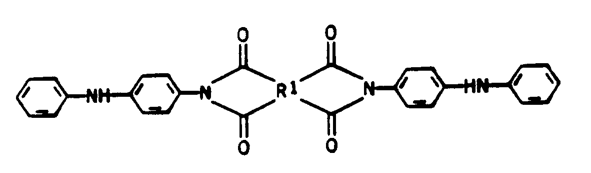 dans laquelle R¹ a la signification donnée dans la revendication 1, a l'exception, pour R¹, de la structure

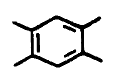

5. Procédé de préparation de polyamide-imides selon la revendication 1, caractérisé en ce qu'un bis(phtalimide) correspondant est polycondensé avec un dichlorure d'acide dicarboxylique correspondant dans un solvant aprotique polaire à une température comprise entre 120 et 300°C.

6. Procédé de préparation de polyamide-imides selon la revendication 1, caractérisé en ce qu'en un procédé dans un seul récipient, une 4-aminophénylalkylamine ou une 4-aminophénylarylamine est mise en réaction avec un dianhydride d'acide tétracarboxylique correspondant dans un solvant aprotique polaire et le produit intermédiaire obtenu est polycondensé avec un dichlorure d'acide dicarboxylique correspondant, la température de réaction se situant entre 120 et 300°C.

7. Procédé selon la revendication 5, caractérisé en ce que la polycondensation est menée en présence d'une base du groupe des amines tertiaires.
